# EUROPÄISCHE PATENTSCHRIFT

(11) **EP 2 806 916 B1**
(45) Veröffentlichungstag und Bekanntmachung des Hinweises auf die Patenterteilung: **07.12.2016**
(21) Anmeldenummer: 14718956.7
(22) Anmeldetag: 16.04.2014
(51) Int. Cl.: A61M 1/36, F16K 11/00, A61M 39/22

(54) **BEFÜLL- UND REZIRKULATIONSVORRICHTUNG EINES FLÜSSIGKEITSSYSTEMS EINES EXTRAKORPORALEN BLUTBEHANDLUNGSGERÄTS**
FILLING AND RECIRCULATION DEVICE FOR A LIQUID SYSTEM OF AN EXTRACORPOREAL BLOOD TREATMENT DEVICE
DISPOSITIF DE REMPLISSAGE ET DE RECIRCULATION D'UN SYSTÈME DE FLUIDE D'UN APPAREIL DE TRAITEMENT SANGUIN EXTRACORPOREL

(30) Priorität: 19.04.2013 DE 102013103986
(43) Veröffentlichungstag der Anmeldung: 03.12.2014
(73) Patentinhaber: B. Braun Avitum AG, 34212 Melsungen (DE)
(72) Erfinder: EIKELMANN, Guido, 34225 Baunatal (DE); HECTOR, Rainer, Dr., 49082 Osnabrück (DE)
(74) Vertreter: Winter, Brandl, Fürniss, Hübner, Röss, Kaiser, Polte - Partnerschaft mbB
(86) Internationale Anmeldenummer: PCT/EP2014/057744
(87) Internationale Veröffentlichungsnummer: WO 2014/170378

(56) Entgegenhaltungen:
- EP-A1- 2 158 934
- US-A- 4 397 335
- US-A- 5 540 653
- US-A1- 2003 125 673

## Beschreibung

Die vorliegende Erfindung betrifft die Befüll- und Rezirkulationsvorrichtung eines Fluidleitungssystems bzw. Flüssigkeitssystems, insbesondere eines extrakorporalen Blutbehandlungsgeräts beispielsweise einer Dialyse- oder Apheresemaschine gemäß dem Oberbegriff des Anspruchs 1.

### Hintergrund der Erfindung

Das hydraulische System (blut-seitiges Flüssigkeitssystem) eines Blutbehandlungsgeräts, beispielsweise einer Dialysemaschine, muss vor Anschluss an einen Patienten mit einer Flüssigkeit, beispielsweise einer NaCl- Lösung oder einer anderen sterilen physiologischen Lösung, gefüllt werden, derart, dass Lufteinschlüsse in dem System entlüftet werden, die für einen, an das System flüssigkeits - angeschlossenen Patienten gefährlich sein würden. Des Weiteren sollte das hydraulische System mit der eingefüllten Flüssigkeit über einen bestimmten Zeitraum durchgespült werden, um im System möglicherweise abgelagerte Schadstoffe, Schmutzpartikel, etc. auszufiltern/auszuschwemmen, bevor das System am Patienten angelegt wird. Diese beiden Vorgänge werden bei einem extrakorporalen Blutbehandlungsgerät im Rahmen eines Befüll-Zirkulations-Zyklus durchgeführt, wieder auch Gegenstand der vorliegenden Erfindung ist.

### Stand der Technik

Im Stand der Technik existieren Flüssigkeitsbehälter vorzugsweise in Form von Kunststoffbeuteln, die speziell für extrakorporale Blutbehandlungsgeräte dieser einschlägigen Gattung konstruiert sind, um die u. a. wie vorstehend definierten Gerätefunktionen zu ermöglichen. Solche Flüssigkeitsbehälter werden auch von der Anmelderin der vorliegenden Anmeldung hergestellt und vertrieben.

Ein solcher Flüssigkeitsbehälter hat in der Regel eine Ftüssigkeitsaufnahmekammer und zwei vorzugsweise verschließbare Flüssigkeitsanschlüsse. An einen ersten der zwei Anschlüsse ist ein arterieller Leitungsabschnitt und an den zweiten Anschluss ein venöser Leitungsabschnitt des hydraulischen Systems (Fluidsysterüs oder auch als Fluidleitungssystem bezeichnet) des extrakorporalen Blutbehandlungsgeräts anschließbar. Der Flüssigkeitsbeutel sowie die beiden Leitungsabschnitte bilden zusammen eine Zirkulationsvorrichtung des extrakorporalen Blutbehandlungsgeräts.

Für den Fluidsystem-Befüllvorgang wird zunächst der arterielle Leitungsabschnitt an den ersten Flüssigkeitsanschluss des Beutels angeschlossen und nach Öffnen des ersten Flüssigkeitsanschlusses das hydraulische System gefüllt. Dabei verbleibt der venöse Leitungsabschnitt des Systems zunächst zur Atmosphäre hin offen oder ist an einen Ablauf, ein Behältnis oder einen Beutel angeschlossen, sodass im System befindliche Luft zur Atmosphäre hin entweichen/entlüftet werden kann. Sobald der Befüllvorgang abgeschlossen ist, wird der venöse Leitungsabschnitt an den zweiten Flüssigkeitsanschluss des Beutels durch Umkonnektieren angeschlossen, um dann die im hydraulischen System des extrakorporalen Blutbehandlungsgeräts befindliche Flüssigkeit für eine bestimmte Zeitspanne oder um ein bestimmtes Strömungsvölumen über die Beutelkammer zu zirkulieren.

Bei diesem optionalen Zirkulationsvorgang durchströmt die Flüssigkeit systeminterne Filtereinrichtungen, in denen restliche Lufteinschlüsse mit der Flüssigkeit entfernt/ausgefiltert werden. Bei Bedarf kann der venöse Leitungsabschnitt des hydraulischen Systems wieder vom zweiten Flüssigkeitsanschluss des Fluidbeutels abgeklemmt und die im hydraulischen System befindliche Flüssigkeit unter ständiger Zufuhr von Flüssigkeit aus dem Behälter nochmals ausgespült werden. In der, Regel wird jedoch der arterielle Leitungsabschnitt vom Flüssigkeitsbeutel abgetrennt und an den Patienten angeschlossen so dass durch das Blut die im Blutschlauchsystem befindliche Flüssigkeit verdrängt und abgeleitet werden kann. Anschließend kann der venöse Leitungsabschnitt an den Patienten angeschlossen werden. Mit dem

Mit Beendigung des Zirkulationsvorgangs und dem Anlegen der beiden Leitungsabschnitte (venös und arteriell) an den Patienten für die Behandlung ist der eine Patientenbehandlung vorbereitende Befüll-/Zirkülationszyklus abgeschlossen.

Aus der vorstehenden Beschreibung des Befüll-/Zirkulationszyklus eines aus dem Stand der Technik bekannten hydraulischen Systems/Flüssigkeitsleitungssystems eines extrakorporalen Blutbehandlungsgeräts (Dialysemaschine) wird ersichtlich, dass für die Befüll- und Zirkulationsvorgänge der Fluidbeutel im Systemkreis verbleibt, d. h. die im System befindliche Flüssigkeit durch den Fluidbeutel bzw. durch dessen Fluidkammer zirkuliert wird. Damit kann möglicherweise die im Fluidbeutel bevorratete Flüssigkeit kontaminiert werden. Dies hat zur Folge, dass der Fluidbeutel in jedem Fall durch einen neuen Fluidbeutel ersetzt werden muss und nicht für eine spätere Therapiephase, z.B. bei der Reinfusion, eingesetzt werden kann. Der Fluidbeutel für den zuvor ausgeführten Befüll-/Zirkulationszyklus wird somit entsorgt, unabhängig von dessen Restfüllstand. Es liegt auf der Hand, dass bei dieser Vorgehensweise im Fall einer hohen Behandlungszahl ein mengenmäßig großer Teil an Flüssigkeit verschwendet wird, da der Flüssigkeitsinhalt eines Flüssigkeitsbeutels nur für den Befüll-/Zirkulatio,nszyklus (unvollständig) verwendbar ist.

Des Weiteren sind Fluidbeutel mit zwei Anschlüssen als Sonderanfertigung teurer als konventionelle Fluidbeutel, beispielsweise NaCl-Beutel/Flaschen, mit nur einem einzigen Anschluss. Daher bedeutet die vorstehend beschriebene Befüll- und Rezirkulationstechnik nicht nur eine Verschwendung an Befüll- und Spülflüssigkeit sondern auch eine Kostensteigerung infolge teurer Einweg-Fluidbeutel.

Schließlich müssen die Leitungen des zu befüllenden extrakorporalen Fluidleitungssystems des Blutbehandlungsgeräts für die einzelnen Befüll- und Rezirkulatiosschritte permanent umgesteckt werden. In anderen Worten ausgedrückt muss die arterielle Leitung zunächst an den Fluidbehälter angekoppelt und die venöse Leitung abgekoppelt sein, um spülen zu können. Dann wiederum muss die venöse Leitung an den Fluidbehälter angekoppelt werden, um rezirkulieren zu können. Für den abschließenden weiteren Spülvorgang wird die venöse Leitung wieder abgekoppelt. Sollen schließlich die arterielle und venöse Leitung an den Patienten angelegt werden, müssen beide Fluidanschlüsse am Fluidbehälter geschlossen werden, um ein unkontrolliertes Auslaufen des Fluidbehälters zu vermeiden. Dies zeigt, dass die Handhabung des bekannten Befüll- und Rezirkulationssstems nicht sonderlich bedienungsfreundlich ist.

Angesichts dieser Problematik ist es die Aufgabe der vorliegenden Erfindung, eine gattungsgemäße Befüll- und Rezirkulationsvorrichtung eines Fluidsystems vorzugsweise eines extrakorporalen Blutbehandlungsgeräts sowie ein Befüll- und Rezirkulationsverfahren bereit zu stellen, was gegenüber dem Stand der Technik wirtschaftlicher und damit kostengünstiger betrieben werden kann. Des Weiteren ist ein Ziel der vorliegenden Erfindung, ein mit der erfindungsgemäßen Befüll- und Rezirkulationsvorrichtung ausgerüstetes Fluidsystem beispielsweise eines extrakorporalen Blutbehandlungsgeräts bereit zu stellen, das in einfacher Weise bedient werden kann und die Verwendung konventioneller Fluidflaschen, (z.B. NaCl-Flaschen) erlaubt um dadurch auch ein Fehlerrisiko bei der Bedienung der Vorrichtung zu senken.

Die US 4397335 A offenbart ein Drehventil, insbesondere zur Verwendung in medizinischen Systemen. Dieses Drehventil hat eine Anordnung von Durchgängen in seinem Drehventilkegel und Ports in seinem Gehäuse, um eine manuelle Betätigung des Ventils zu ermöglichen, um die medizinischen Systems zur Überwachung des Pulmonalarteriendrucks und zur Messung des zentralen Venendrucks umzuschalten. Das System kann auch für die Messung der Herzleistung oder zur Entnahme von gemischten venösen Blut verwendet werden. Patienten-Traumata und Verunreinigungen können so vermieden werden.

Diese Aufgabe wird durch eine Befüll- und Rezirkulationsvorrichtung eines Fluidsystems/Flüssigkeitssystems vorzugsweise eines extrakorporalen Blutbehandlungsgeräts (Dialyse-, Apheresemaschine) mit den Merkmalen des Anspruchs 1 gelöst. Ferner wird die Aufgabe durch ein Verfahren mit den Merkmalen des Anspruchs 6 und/oder 11 gelöst. Vorteilhafte Ausgestaltungen der Erfindung sind Gegenstand der Unteransprüche

Der Grundgedanke der vorliegenden Erfindung besteht darin, die gattungsgemäße Befüllvorrichtung eines Fluidleitungssystems insbesondere eines extrakorporalen Blutbehandlungsgeräts so auszubilden, dass ein konventioneller (preisgünstiger) Fluidbehälter bekannter Bauart, etwa eine herkömmliche NaCl-Flasche oder ein entsprechender Beutel mit einem einzigen punktierbaren/konnektierbaren Verschluss verwendet werden kann. Der Anschluss dieses Fluidbehälters erfolgt über eine einzige manuell betätigare Fluid-Sperreinrichtung, mittels der alle Befüll- und (Re-)Zirkulationsvorgänge schaltbar sind, so dass die arteriellen und venösen Leitungen des exrakorporalen Leitungssystems jeweils nur einmal (zum Anschließen des Patienten) pro Therapie umkonnektiert werden müssen.

Demzufolge wird eine Befüll- und (Re-)Zirkulationsvorrichtung eines Fluidleitungssystems eines extrakorporalen Blutbehandlungsgeräts vorgeschlagen, die einen Fluidbehälteranschluss und eine manuell betätigbare Fluid-Sperreinrichtung in Form eines dreidimensionalen Vier-Wege-Ventils hat mit einer ersten Ventilebene, in der ein Einlassanschluss für den Fluidbehälter, ein arterieller Leitungsanschluss, sowie ein Ventil-interner Verbindungsanschluss angeordnet sind, mit einer zweite Ventilebene, in der ein Auslassanschluss sowie ein venöser Leitungsanschluss angeordnet sind, der mit dem Ventil-internen Verbindungsanschluss der ersten Ebene permanent fluidverbunden ist und mit einem manuell zu betätigenden Ventil-Drehkolben, der einen ersten, der ersten Ventilebene zugeordneten Kolben-Regelabschnitt und einen axial beabstandeten zweiten, der zweiten Ventilebene zugeordneten Kolben-Regelabschnitt hat, die so aufeinander abgestimmt sind, dass
a. in einer Ventilkolben-Drehposition für Spülen der erste Kolben-Regelabschnitt den Einlassanschluss mit dem arteriellen Leitungsanschluss und der zweite Kolben-Regelabschnitt den venösen Leitungsanschluss mit dem Auslassanschluss verbindet,
b. in einer Ventilkolben-Drehposition für (Re-)Zirkulieren der erste Kolben-Regelabschnitt den Ventil-internen Verbindungsanschluss mit dem arteriellen Anschluss und der zweite Kolben-Regelabschnitt den Auslassanschluss vom venösen Ventilanschluss trennt und
c. in einer Ventilkolben-Drehposition für Therapieren der erste und der zweite Kolben-Regelabschnitt alle Anschlüsse sperrt.

Durch die Anordnung der erfindungsgemäßen Fluid-Sperreinrichtung am Fluidbehälteranschluss kann dieser an dem konventionellen medizinischen Fluidbehälter, vorzugsweise nach Punktieren des Behälterverschlusses, verbleiben, wohingegen die arteriellen und venösen Leitungsabschnitte an der Fluid-Sperreinrichtung für einen Befüll- und (Re-)Zirkulationsvorgang des Fluidleitungssystems angeschlossen bleiben und erst danach wieder von der Fluid-Sperreinrichtung abklemmbar sind, ohne dass Fluid aus dem medizinische Fluidbehälter verloren geht. Ein Umkonnektieren der arteriellen und venösen Leitungen zwischen dem Befüll- und (Re-)Zirkulationsvorgang entfällt somit.

Da der arterielle und venöse Leitungsabschnitt ferner von den Anschlüssen der Fluid-Sperreinrichtung (z.B. Luer-Lock-Anschluss) abkoppelbar sind, können der arterielle und der venöse Leitungsabschnitt unmittelbar nach Befüllen und (Re-)Zirkulieren an einen arteriellen und venösen Patientenzugang angeschossen werden, ohne dass am arteriellen und venösen Leitungsabschnitt bzw. deren Anschlüsse irgend welche Veränderungen vorgenommen werden müssen. Dadurch wird die Handhabung der erfindungsgemäßen Befüll- und (Re-)Zirkulationsvorrichtung vereinfacht.

Die Erfindung wird nachstehend anhand eines bevorzugten Ausführungsbeispiels unter Bezugnahme auf die begleitenden Figuren näher erläutert.
Fig. 1 zeigt ein Fluidleitungssystem vorzugsweise in Form einer Zirkulationsvorrichtung eines extrakorporalen Blutbehandlungsgeräts (Dialysemaschine) gemäß einem bevorzugten Ausführungsbeispiel der Erfindung,
Fig. 2 zeigt die manuell zu betätigende Fluid-Sperreinrichtung gemäß der vorliegenden Erfindung als Prinzipdarstellung,
Fig. 3a bis 3e zeigen die Schaltstellungen der erfindungsgemäßen Fluid-Sperreinrichtung für die einzelnen Vorgänge, insbesondere Befüll-, (Re-)Zirkulations- und Therapievorgänge sowie optional Patientenkonnektierungs- und Leitungsentleerungsvorgänge und
Fig. 4a und 4b zeigen alternative Schaltstellungen der erfindungsgemäßen Fluid-Sperreinrichtung für die einzelnen Vorgänge "Befüllen" und "(Re-)Zirkuliereh".

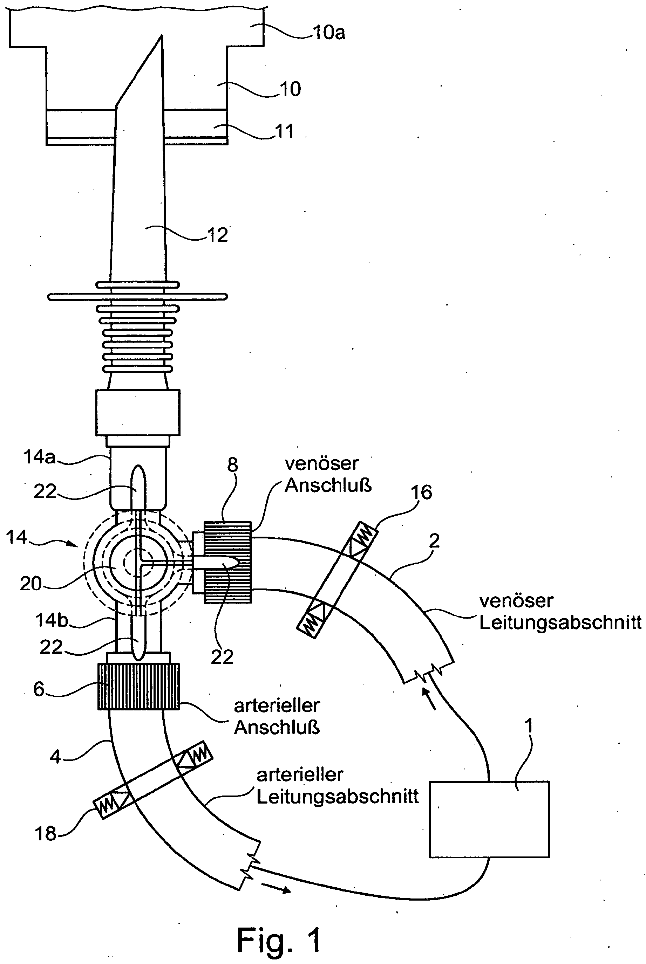

Gemäß der Fig. 1 hat ein extrakorporales Blutbehandlungsgerät 1, beispielsweise eine Dialyse- oder Apheresemaschine ein hydraulisches Leitungssystem (nachfolgend als Fluidleifiungssystem bezeichnet), durch das während einer Behandlungsphase auf der Maschinenseite beispielsweise eine Blut - Reinigungsflüssigkeit (Dialysierflüssigkeit) geleitet wird und auf der Patientenseite Blut extrakorporal hindurchfließt, wobei das maschinenseitige und patientenseitige Fluidleitungssystem im Fall einer Dialysemaschine durch einen nicht weiter dargestellten Dialysator (Filter) fluidisch getrennt sind.

Hierfür hat das (blutführende) Fluidleitungssystem patientenseitig einen venösen sowie einen arteriellen Leitungsabschnitt 2, 4, vorzugsweise jeweils Schlauchabschnitt mit jeweils endseitig angeordnete/ ausgebildeten Anschlüssen (Luer-Lock-Anschlüssen) 6, 8, an die beispielsweise Injektionsnadeln oder Kanülen (nicht dargestellt) als Patientenzugang anschließbar sind, welche in einen Patientenkörper einführbar sind.

Um ein möglicherweise notwendiges Ausschwemmen von ggf. herstellungsbedingten Verunreinigungen in den Patientenkörper zu vermeiden, hat das extrakorporale Blutbehandlungsgerät 1 eine Befüll- und (Re-)Zirkulätionsvorrichtung, welche vorliegend einen Befüll- und Zirkulationsprozess ermöglicht, wodurch das patientenseitige Fluidleitungssystem in der Regel vor jeder Patientenbehandlung gereinigt wird.

Gemäß der Fig. 1 hat die Befüll-/Rezirkulationsvorrichtung (oder auch Befüll-/Zirkulationsvorrichtung) gemäß der vorliegenden Erfindung eine Flüssigkeitsquelle vorzugsweise in Form eines universellen Flüssigkeitsbehälters (NaCl-FlaschelBeutel) 10 mit einem einzigen Auslass 11, der beim vorliegenden Ausführungsbeispiel mittels eines Spike 12 als Fluidbehälteranschluss der Befüll-/Rezirkulationsvorrichtung punktiert wird, um Flüssigkeit aus dem Flüssigkeitsbehälter 10 abzuzapfen. Der Aufbau des Spike 12 entspricht bekannten Spikekonstruktionen, sodass an dieser Stelle auf dessen Aufbau nicht weiter eingegangen werden muss. Ferner sei darauf hingewiesen, dass beispielsweise im Fall eines Luer-Lock- oder andersartigen Anschlusses auf der Behälterseite der Spike durch ein entsprechendes Anschlussstück auf Seiten der Befüll-/Rezirkulationsvorrichtung ersetzt sein kann. Des Weiteren hat die erfindungsgemäße Befüll-/Rezirkulationsvorrichtung eine Fluid-Sperreinrichtung vorliegend in Form eines dreidimensionalen 4-Wege - Ventils oder auch 4-Wege- Hahns 14, der dem Spike 12 (Anschlussstück) in Strömungsrichtung weg vom Flüssigkeitsbehälter 10 gesehen, (unmittelbar) nachgeschaltet ist.

Im vorliegenden Fall ist der Spike 12 direkt (ohne Zwischenschaltung eines zusätzlichen Leitungsstücks) an das 4-Wege-Ventil 14 angeschlossen. Alternativ kann der Spike 12 auch einstückig bzw. als eine Baueinheit mit dem 4-Wege-Ventil 14 ausgebildet sein. Auch kann der Spike 12 über ein Zwischenleitungsstück an das Ventil 14 angeschlossen sein, wobei zwischen Spike und Ventil ein zusätzliches Rückschlagventil angeordnet sein kann, welches eine Strömung in Richtung zum Fluidbehälter 10 sperrt.

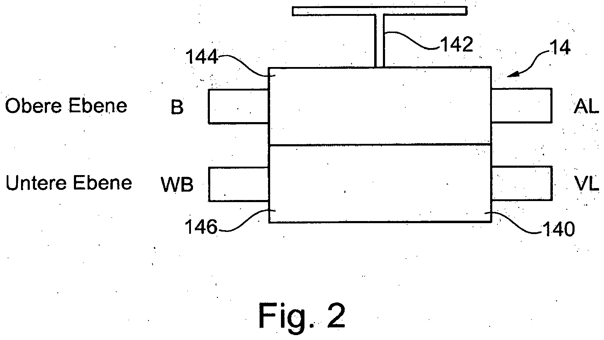

Das dreidimensionale 4-Wege-Ventil 14 gemäß der Fig. 2 hat ein zylindrisches Gehäuse 140, in das ein manuell drehbarer Ventilkolben 142 axial eingesetzt ist. Das Ventilgehäuse 140 bildet in einem oberen axialen Ventilabschnitt 144 eine erste Ventilebene aus, in der ein Einlassanschluss B für den Fluidbehälter 10 sowie ein arterieller Leitungsanschluss AL ausgeformt sind, die in Umfangsrichtung des Ventilgehäuses 140 beabstandet, vorzugsweise radial gegenüberliegend angeordnet sind. Des Weiteren bildet das Ventilgehäuse 140 in einem unteren axialen Ventilabschnitt 146 eine zweite Ventilebene aus, in der ein Auslassanschluss WB sowie ein venöser Leitungsanschluss VL angeordnet sind, die ebenfalls in Umfangsrichtung beabstandet, vorzugsweise radial gegenüberliegend angeordnet sind. Dabei sind die Anschlüsse B, AL der ersten Ventilebene 144 zu den Anschlüssen WB, VL der zweiten Ventilebene 146 in Umfangsrichtung versetzt angeordnet.

In der ersten Ventilebene 144 ist ferner ein Ventil-interner Verbindungsanschluss VI ausgebildet, der über einen nicht weiter gezeigten Axialkanal innerhalb des Ventilgehäuses 140 mit dem venösen Leitungsanschluss VL der zweiten Ventilebene 146 permanent fluidverbunden ist.

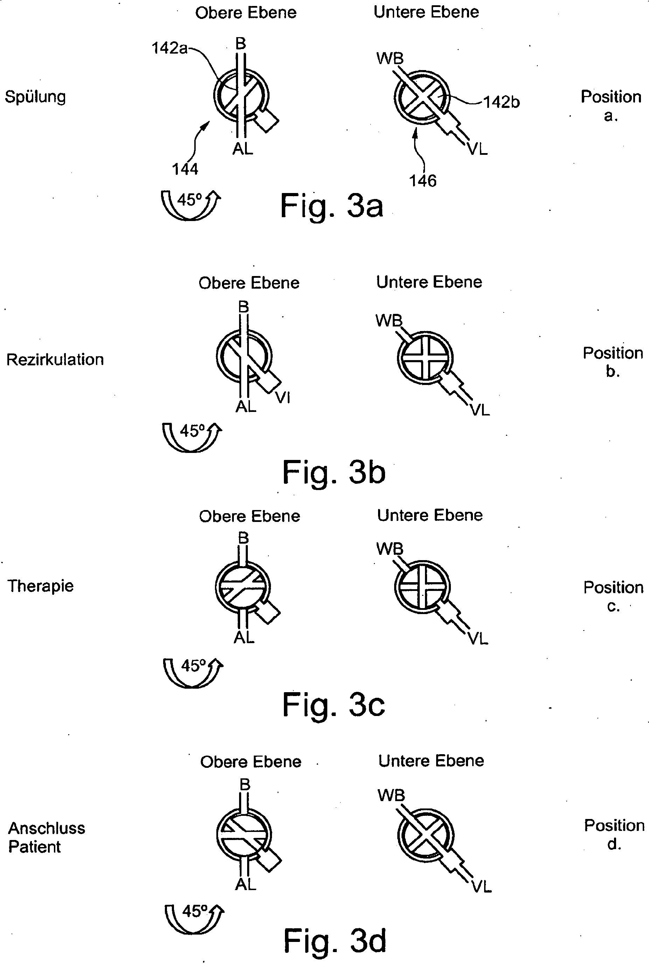

Schließlich weist der manuell zu betätigende Ventil-Drehkolben 142 einen, ersten, der ersten Ventilebene 144 zugeordneten axialen Kolben-Regelabschnitt 142a und einen axial hiervon beabstandeten zweiten, der zweiten Ventilebene 146 zugeordneten Kolben-Regelabschnitt 142b auf. In beiden Kolben-Regelabschnitten 142a, b sind zwei sich kreuzende Radial-Durchgangsbohrungen ausgeformt, deren Verläufe und Ausrichtungen so aufeinander abgestimmt sind, dass in einer Ventilkolben-Drehposition für Spülen gemäß der Fig. 3a der erste Kolben-Regelabschnitt 142a den Einlassanschluss B mit dem arteriellen Leitungsanschluss VA und der zweite Kolben-Regelabschnitt 142b den venösen Leitungsanschluss VL mit dem Auslassanschluss WB verbindet; in einer Ventilkolben-Drehposition für (Re-)Zirkulieren gemäß der Fig. 3b der erste Kolben-Regelabschnitt 142a den Ventil-internen Verbindungsanschluss VI mit dem arteriellen Leitungsanschluss AL und der zweite Kolben-Regelabschnitt 14ab den Auslassanschluss WB vom venösen Leitungsanschluss VL trennt und in einer Ventilkolben-Drehposition für Therapieren gemäß der Fig. 3c der erste und der zweite Kolben-Regelabschnitt 142a, b alle Anschlüsse sperrt.

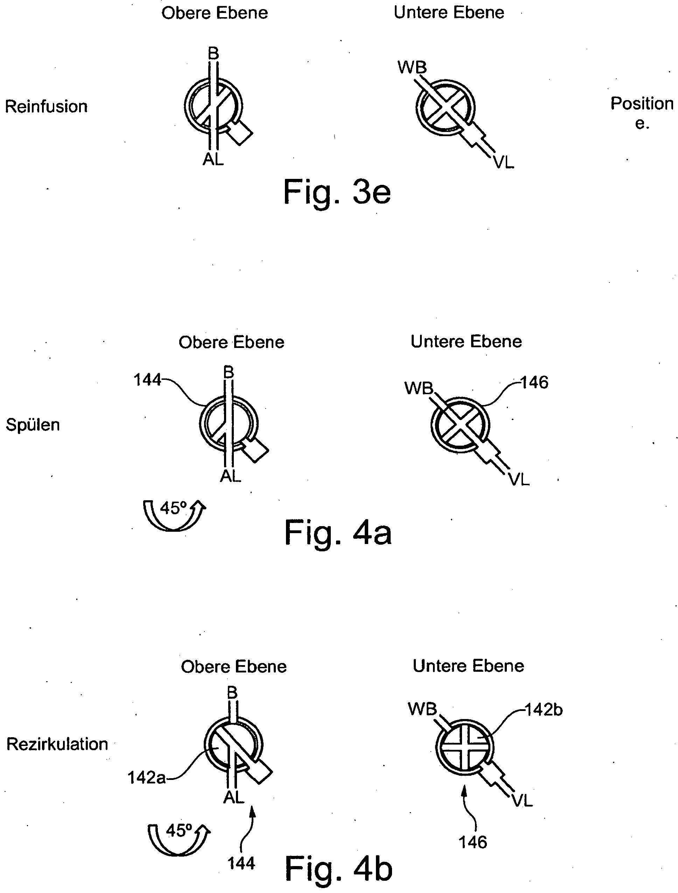

Des Weiteren ermöglichen die beiden Kolben-Regelabschnitte 142a, b zumindest zwei weitere Ventilkolben-Drehpositionen nämlich eine Ventil-Drehposition für den Vorgang des Anschließens der arteriellen und venösen Leitungen 2,4 an einen Patienten gemäß der Fig. 3d, in welcher der erste Kolben-Regelabschnitt 142a den Einlassanschluss B vom arteriellen Leitungsanschluss AL trennt und der zweite Kolben-Regelabschnitt 142b den venösen Leitungsanschluss VL mit dem Auslassanschluss WB verbindet und eine Ventil-Drehposition für den Vorgang des Blutausdrückens aus den arteriellen und venösen Leitungen 2, 4 in einen Patienten gemäß der Fig. 3e, in welcher der erste Kolben-Regelabschnitt 142a den Einlassanschluss B mit dem arteriellen Leitungsanschluss AL verbindet und der zweite Kolben-Regelabschnitt 142b den venösen Leitungsanschluss VL mit dem Auslassanschluss WB verbindet.

Schließlich sind an den arteriellen und venösen Leitungsabschnitten 2, 4 gemäß der Fig. 1 noch Schlauchklemmen 16, 18 angeordnet, die insbesondere für den Vorgang des Anschließens der blutseitigen Leitungssystems des Blutbehandlungsgeräts an den Patienten betätigbar sind, um die arteriellen und venösen Leitungen 2, 4 zu sperren und damit das darin enthaltene Fluid auch nach Abkoppeln vom 4-Wege-Ventil 14 zu speichern.

Die Funktion der erfindungsgemäßen Befüll-/Rezirkulationsvorrichtung wird nachfölgend anhand der Fig. 3a-e erläutert:
Zunächst verharrt das 4-Wege,-Ventil 14 in einer Ventil-Sperrstellung beispielsweise gemäß der Fig. 3c oder 3e, in welcher zumindest der Einlassanschluss B in der ersten Ventilebene 142a gesperrt ist. In dieser Stellung kann das Ventil 14 an den Fluidbehälters 10 angeschlossen werden, ohne dass Fluid aus dem Behälter 10 unkontrolliert ausläuft.

Hierauf wird der Ventil-Drehkolben 142 in die Ventil-Drehposition gemäß-der Fig. 3a gedreht (etwa um 45°), wodurch die zuvor angeschlossenen arteriellen und venösen Leitungen 2, 4 seriell mit dem Fluidbehälter 10 verbunden sind und mit Fluid befüllt werden können. Da jedoch die venöse Leitung 2 in dieser Ventilstellurig mit dem Auslass WB des Ventils 14 verbunden ist, wird eine bestimmte Fluidmenge durch die Leitungen 2, 4 gespült und nimmt dabei darin enthaltene Verunreinigungen und Lufteinschlüsse mit.

Anschließend wird der Ventil-Drehkolben 142 in die Position gemäß der Fig. 3b weitergedreht (um ca. 45°), in welcher die arteriellen und venösen Leitungen 2, 4 über den Ventil-internen Verbindungsanschluss VI und den Axialkanal kurzgeschlossen werden und dadurch ein (Re-)Zirkulationskreislauf gebildet wird.

An dieser Stelle sei darauf hingewiesen, dass in dieser Ventilstellung der Fluidbehälter 10 nach wie vor an das Leitungssystem angeschlossen ist, um ggf. Fluid nachzufördern. Alternativ hierzu kann es aber gemäß der Fig. 4a und 4b vorgesehen sein, dass bei Weiterdrehen des Ventil-Drehkolbens 142 aus der Befüllposition (siehe Fig. 4a) in die Rezirkulationsposition (siehe Fig. 4b) der Fluidbehälter vom Leitungssystem abgekoppelt wird, um eine Kontamination des Behälterinhalts in jedem Fall zu vermeiden.

Nach der Rezirkulation wird der Ventil-Drehkolben 142 in eine Position gemäß der Fig. 3d weitergedreht (um ca. 45°), in welcher der Fluidbehälter 10 abgekoppelt ist. In dieser Stellung kann der venöse Leitungsabschnitt 2 mit dem Ventilauslassanschluss WB verbunden bleiben, da zuvor die Schlauchklemmen 16, 18 betätigt worden sind, um das eingefüllte Fluid in den Leitungen 2, 4 zu halten. Nunmehr können die arteriellen und venösen Leitungen 2, 4 vom 4-Wege-Ventil 14 abgeklemmt und an den Patienten angeschlossen werden. Dabei kann zunächst nur die arterielle Leitung an den Patienten angeschlossen werden, so dass in der Fig. 3d gezeigte Position durch Blut die Flüssigkeit aus dem Blutschlauchsystem verdrängt und über den Auslassanschluss WB abgeleitet wird, bevor anschließend die venöse Leitung an den Patienten angeschlossen wird. Darauf hin wird der Ventil-Drehkolben 142 in die Position gemäß der Fig. 3c weitergedreht (um ca. 45°), in der alle Anschlüsse des 4-Wege-Ventils gesperrt sind.

Nach der Therapie befindet sich noch Patientenblut in dem extrakorporalen Leitungssystem, das dem Patienten wieder zugeführt werden muss. Zu diesem Zweck wird zunächst nur die arterielle Leitung 3 erneut an das noch gesperrte 4-Wege-Ventil 14 angeschlossen und der Ventil-Drehkolben 142 in die Drehposition gemäß der Fig. 3e weitergedreht (um ca. 45°), wodurch der Fluidbehälter 10 mit der arteriellen Leitung 4 verbunden und das in den arteriellen und venösen Leitungen 2, 4 stehende Blut in den Patientenkörper rückgeführt wird. Damit ist der Gesamtvorgang beendet.

## Patentansprüche

1. Befüll- und Rezirkulationsvorrichtung eines Fluidleitungssystems eines extrakorporalen Blutbehandlungsgeräts (1), die folgende Elemente hat:
- einen Fluidbehälternschluss (12), vorzugsweise in Form eines Spike, der für das Konnektieren des einzigen Fluidanschlusses (11) eines medizinischen Fluidbehälters (10) angepasst ist und
- eine manuell zu betätigende Fluid-Sperreinrichtung (14), die stromab zu dem Fluidbehälteranschluss (12) angeordnet und dazu angepasst oder dafür vorgesehen ist, für den gesamten Betrieb der Befüll- und Rezirklationsvorrichtung über den Fluidbehälteranschluss (12) mit dem Fluidbehälter (10) fluidverbunden zu bleiben, **dadurch gekennzeichnet, dass**
- die Fluid-Sperreinrichtung (14) ein dreidimensionales Vier-Wege-Ventil ist mit einer ersten Ventilebene (144), in der ein Einlassanschluss (B) für den Fluidbehälter (10), ein arterieller Leitungsanschluss (AL), sowie ein Ventil-interner Verbindungsanschluss (VI) angeordnet sind,'mit einer zweite Ventilebene (146), in der ein Auslassanschluss (WB) sowie ein venöser Leitungsanschluss (VL) angeordnet sind, der mit dem Ventil-internen Verbindungsanschluss (VI) der ersten Ebene (144) permanent fluidverbunden ist und mit einem manuell zu betätigenden Ventil-Drehkolben (142), der einen ersten, der ersten Ventilebene (144) zugeordneten Kolben-Regelabschnitt (142a) und einen axial beabstandeten zweiten, der zweiten Ventilebene (146) zugeordneten Kolben-Regelabschnitt (142b) hat, die so aufeinander abgestimmt sind, dass
a. in einer Ventilkolben-Drehposition für Spülen der erste Kolben-Regelabschnitt (142a) den Einlassanschluss (B) mit dem arteriellen Leitungsanschluss (VL) und der zweite Kolben-Regelabschnitt (142b) den venösen Leitungsanschluss (VL) mit dem Auslassanschluss (WB) verbindet,
b. in einer Ventilkolben-Drehposition für Rezirkulieren der erste Kolben-Regelabschnitt (142a) den Ventil-internen Verbindungsanschluss (VI) mit dem arteriellen Anschluss (AL) und der zweite Kolben-Regelabschnitt (142b) den Auslassanschluss (WB) vom venösen Leitungsanschluss (VL) trennt und
c. in einer Ventilkolben-Drehposition für Therapieren der erste und der zweite Kolben-Regelabschnitt (142a, b) alle Anschlüsse sperrt.

2. Befüll- und Rezirkulationsvorrichtung nach Anspruch 1, **dadurch gekennzeichnet, dass**
d. in einer Ventilkolben-Drehposition für Patientenkonnektieren vorzugsweise in Drehrichtung zwischen den Ventilpositionen gemäß b. und c. der erste Kolben-Regelabschnitt (142a) den Einlassanschluss (B) und den arteriellen. Anschluss (AL) sperrt und der zweite Kolben-Regelabschnitt (142b) den venösen Leitungsanschluss (VL) mit dem Auslassanschluss (WB) verbindet.

3. Befüll- und Rezirkulationsvorrichtung nach Anspruch 1 oder 2, **dadurch**
**gekennzeichnet, dass**
ein einer Ventilkolben-Drehposition für Reinfusion vorzugsweise in Drehrichtung nach der Ventilposition gemäß c. der erste Kolben-Regelabschnitt (142a) den Einlassanschluss (B) mit dem arteriellen Leitungsanschluss (AL) verbindet und der zweite Kolben-Regelabschnitt (142b) den venösen Leitungsanschluss (VL) mit dem Auslassanschluss (WB) verbindet.

4. Befüll- und Rezirkulationsvorrichtung nach einem der vorstehenden Ansprüche, **dadurch gekennzeichnet, dass** in der Ventil-Drehposition gemäß, b. der erste Kolben-Regelabschnitt (142a) den Einlassanschluss (B) mit dem arteriellen Leitungsanschluss (AL) verbindet.

5. Befüll- und Rezirkulationsvorrichtung nach einem der vorstehenden Ansprüche 1 bis 3, **dadurch gekennzeichnet, dass** in der Ventil-Drehposition gemäß b. der erste Kolben-Regelabschnitt (142a) den Einlassanschluss (B) vom arteriellen Leitungsanschluss (AL) trennt.

6. Verfahren zur Durchführung eines Befüll- und Rezirkulationsvorgangs eines blutseitigen, Leitungssystems einer Dialysemaschine unter Verwendung einer Befüll- und Rezirkulationsvorrichtung nach einem der Ansprüche 1 bis 5, mit den Schritten:
S1. Anschließen des Fluidbehälteranschlusses (12) an den einzigen Fluidanschluss (11) des medizinischen Fluidbehälters (10) bei vollständig gesperrtem Vier-Wege-Ventil (14) sowie Anschließen der arteriellen und venösen Leitungsabschnitte (2, 4) an die arteriellen und venösen Leitungsanschlüsse (AL, VL) des noch gesperrten Vier-Wege-Ventils (14),
S2. Durchspülen eines Fluidströmungspfads entlang des arteriellen Fluidleitungsabschnitts (4) und des venösen Fluidleitungsabschnitts (2) des extrakorporalen Blutbehandlungsgeräts (1) durch Drehen des Ventil-Drehkolbens (142) aus einer Ventilsperrstellung in die Ventilkolben-Drehposition a.
S3. Rezirkulieren des Fluidströmungspfads durch Weiterdrehen des Ventil-Drehkolbens (142) aus der Ventilkolben-Drehposition a. in die Ventilkolben-Drehposition b.
S4. Anschließen des Fluidleitungssystems des extrakorporalen Blutbehandlungsgeräts an einen Patienten und Durchführen einer Blutbehandlung nach vorherigem Weiterdrehen des Ventil-Drehkolbens (142) aus der Ventilkolben-Drehposition b. vorzugsweise über die Ventilkolben-Drehposition d. in die Ventil-Drehposition c. und optional
S5. Entleeren des Fluidleitungssystems von Blut durch Weiterdrehen des Ventil-Drehkolbens (142) aus der Ventilkolben-Drehposition c. in die Ventilkolben-Drehposition e.

7. Verfahren zur Durchführung eines Befüll- und Bezirkulationsvorgangs gemäß Anspruch 6, **dadurch gekennzeichnet, dass** im Verfahrensschritt S4 zunächst der arterielle Leitungsabschnitt (2) an den Patienten angeschlossen wird, um im Fluidleitungssystem befindliches Fluid durch Blut zu verdrängen, und anschließend der venöse Leitungsabschnitt (4) an den Patienten angeschlossen wird.

8. Verfahren zur Durchführung eines Befüll- und Rezirkulationsvorgangs gemäß Anspruch 6 oder 7, **dadurch gekennzeichnet, dass** im Verfahrensschritt S4 das Vier-Wege-Ventil (14) vom arteriellen Leitungsabschnitt (2) getrennt wird, nachdem der Ventil-Drehkolben (142) in die Ventil-Drehposition d. weitergedreht wurde.

9. Verfahren zur Durchführung eines Befüll- und Rezirkulationsvorgangs gemäß einem der Ansprüche 6 bis 8, **dadurch gekennzeichnet, dass** im Verfahrensschritt S4 das Vier-Wege-Ventil (14) vom venösen Leitungsabschnitt (4) getrennt wird, nachdem der Ventil-Drehkolben (142) in die Ventil-Drehposition c. weitergedreht wurde.

10. Verfahren zur Durchführung eines Befüll- und Rezirkulationsvorgangs gemäß Anspruch 6, **dadurch gekennzeichnet, dass** im Verfahrensschritt S5 das Vier-Wege-Ventil (14) an das Fluidleitungssystem des extrakorporalen Blutbehandlungsgeräts erneut angeschlossen wurde, bevor der Ventil-Drehkolben (142) in die Ventil-Drehposition e. weitergedreht wird.

11. Verwendung einer Befüll- und Rezirkulationsvorrichtung nach einem der Ansprüche 1 bis 5 zur Befüllung eines blutseitigen Fluidleitungssystems einer Dialysemaschine vorzugsweise nach einem Verfahren gemäß einem der Ansprüche 6 bis 10.

## Claims

1. Filling and recirculation device of a fluid conducting system of an extracorporeal blood treatment device (1) which has the following components:
- a fluid container connector (12), preferably in the form of a spike, that is adapted for connecting up the single fluid connector (11) of a medical fluid container (10), and
- a manually operable fluid blocking mechanism (14) which is arranged downstream of the fluid container connector (12) and is adapted or provided in such a way so as to remain fluidically connected with the fluid container (10) during the entire operation of the filling and recirculation device by way of the fluid container connector (12), **characterised in that**
- the fluid blocking mechanism (14) is a three-dimensional four-way valve with a first valve plane (144) on which an inlet connector (B) for the fluid container (10), an arterial line connector (AL) as well as a valve-internal coupling connector (VI) are arranged, with a second valve plane (146) on which an outlet connector (WB) as well as a venous line connector (VL) are arranged, which is permanently fluidically connected with the valve-internal coupling connector (VI) of the first plane (144), and with a manually operable valve rotary piston (142) which has a first piston control section (142a) assigned to the first valve plane (144) and an axially spaced second piston control section (142b) assigned to the second valve plane (146), which are geared to each other in such a way that
a. in a valve piston rotary position for flushing, the first piston control section (142a) connects the inlet connector (B) with the arterial line connector (VL), and the second piston control section (142b) connects the venous line section (VL) with the outlet connector (WB),
b. in a valve piston rotary position for recirculation, the first piston control section (142a) connects the valve-internal coupling connector (VI) with the arterial connector (AL), and the second piston control section (142b) disconnects the outlet connector (WB) from the venous line connector (VL), and
c. in a valve piston rotary position for therapy, the first and the second piston control sections (142a, b) close off all connectors.

2. Filing and recirculation device according to claim 1, **characterised in that** d. in a valve piston rotary position for patient connecting, preferably in rotational direction between the valve positions according to b. and c., the first piston control section (142a) closes the inlet connector (B) and the arterial connector (AL) and the second piston control section (142b) connects the venous line connector (VL) with the outlet connector (WB).

3. Filling and recirculation device according to claim 1 or 2, **characterised in that** e. in a valve piston rotary position for re-infusion, preferably in rotational direction after the valve position according to c., the first piston control section (142a) connects the inlet connector (B) with the arterial line connector (AL) and the second piston control section (142b) connects the venous line connector (VL) with the outlet connector (WB).

4. Filling and recirculation device according to one of the claims above, **characterised in that** in the valve rotary position according to b., the first piston control section (142a) connects the inlet connector (B) with the arterial line connector (AL).

5. Filling and recirculation device according to one of the claims 1 to 3 above, **characterised in that** in the valve rotary position according to b., the first piston control section (142a) disconnects the inlet connector (B) from the arterial line connector (AL).

6. Procedure for performing a filling and recirculation process of a blood-side conducting system of a dialysis machine using a filling and recirculation device according to one of the claims 1 to 5, with the steps:
S1. Connecting of the fluid container connector (12) to the single fluid connector (11) of the medical fluid container (10) with the four-way valve (14) completely closed, and connecting of the arterial line section and the venous line section (2, 4) to the arterial and venous line connectors (AL, VL) of the four-way valve (14) that is still closed,
S2. Flushing of a fluid flow path along the arterial fluid line section (4) and the venous line section (2) of the extracorporeal blood treatment device (1) by rotating the valve rotary piston (142) from a valve shut-off position in the valve piston rotary position a.
S3. Recirculating of the fluid flow path by further rotating the valve rotary piston (142) from the valve piston rotary position a. in the valve piston rotary position b.
S4. Connecting of the fluid conducting system of the extracorporeal blood treatment device to a patient and performing of a blood treatment after previous further rotating of the valve rotary piston (142) from the valve piston rotary position b., preferably via the valve piston rotary position d., in the valve piston rotary position c., and optionally
S5. Draining of the fluid conducting system of blood by further rotating the valve rotary piston (142) from the valve piston rotary position c. in the valve piston rotary position e.

7. Procedure for performing a filling and recirculation process according to claim 6, **characterised in that** in process step S4, first the arterial line section (2) is connected to the patient in order to have fluid inside the fluid conducting system displaced by blood, and then the venous line section (4) is connected to the patient.

8. Procedure for performing a filling and recirculation process according to claim 6 or 7, **characterised in that** in process step S4, the four-way valve (14) is disconnected from the arterial line section (2) after the valve rotary piston (142) was further rotated in valve rotary position d.

9. Procedure for performing a filling and recirculation process according to one of the claims 6 to 8, **characterised in that** in process step S4, the four-way valve (14) is disconnected from the venous line section (4) after the valve rotary piston (142) was further rotated in valve rotary position c.

10. Procedure for performing a filling and recirculation process according to claim 6, **characterised in that** in process step S5, the four-way valve (14) was again connected to the fluid conducting system of the extracorporeal blood treatment device before the valve rotary piston (142) is further rotated in valve rotary position e.

11. Use of a filling and recirculation device according to one of the claims 1 to 5 to fill a blood-side fluid conducting system of a dialysis machine, preferably on the basis of a procedure according to one of the claims 6 to 10.

## Revendications

1. Dispositif de remplissage et de recirculation d'un système de conduite de fluide d'un appareil de traitement extracorporel du sang (1), présentant les éléments suivants :
- un raccord de récipient de fluide (12), de préférence sous la forme d'une pointe, qui est adaptée pour la connexion du seul raccord de fluide (11) d'un récipient de fluide médical (10) et
- un dispositif de blocage de fluide (14) actionnable manuellement, qui est disposé en aval du raccord de récipient de fluide (12) et qui est adapté ou prévu pour rester en communication fluidique pendant l'ensemble de l'exploitation du dispositif de remplissage et de recirculation par le biais du raccord de récipient de fluide (12) avec le récipient de fluide (10), **caractérisé en ce que**
- le dispositif de blocage de fluide (14) est une vanne à quatre voies tridimensionnelle munie d'un premier plan de vanne (144), dans lequel sont disposés un raccord d'entrée (B) pour le récipient de fluide (10), un raccord de conduite artérielle (AL), ainsi qu'un raccord de communication (VI) interne à la vanne, munie d'un deuxième plan de vanne (146), dans lequel sont disposés un raccord de sortie (WB), ainsi qu'un raccord de conduite veineux (VL), qui est en communication fluidique permanente avec le raccord de communication (VI) interne à la vanne du premier plan (144) et avec un piston rotatif de vanne (142) actionnable manuellement, qui présente une première partie de régulation de piston (142a) associée au premier plan de vanne (144) et une deuxième partie de régulation de piston (142b) associée au deuxième plan de vanne (146), située à une certaine distance axiale, qui sont adaptées l'un sur l'autre de sorte que
a. dans une position de rotation de piston de vanne pour le rinçage, la première partie de régulation de piston (142a) relie le raccord d'entrée (B) au raccord de conduite artériel (VL) et la deuxième partie de régulation de piston (142b) relie le raccord de conduite veineux (VL) au raccord de sortie (WB),
b. dans une position de rotation de piston de vanne pour la recirculation, la première partie de régulation de piston (142a) sépare le raccord de communication (VI) interne à la vanne du raccord artériel (AL) et la deuxième partie de régulation de piston (142b) sépare le raccord de sortie (WB) du raccord de conduite veineux (VL) et
c. dans une position de rotation de piston de vanne pour les traitements, la première et la deuxième partie de régulation de piston (142 a, b) bloquent tous les raccords.

2. Dispositif de remplissage et de recirculation selon la revendication 1, **caractérisé en ce que**
d. dans une position de rotation de piston de vanne pour établir une connexion avec le patient, de préférence dans un sens de rotation entre les positions de vanne selon b. et c., la première partie de régulation de piston (142a) bloque le raccord d'entrée (B) et le raccord artériel (AL) et la deuxième partie de régulation de piston (142b) relie le raccord de conduite veineux (VL) au raccord de sortie (WB).

3. Dispositif de remplissage et de recirculation selon la revendication 1 ou 2, **caractérisé en ce que**
e. dans une position de rotation de piston de vanne pour la reperfusion, de préférence dans le sens de rotation d'après la position de la vanne selon c., la première partie de régulation de piston (142a) relie le raccord d'entrée (B) au raccord de conduite artériel (AL) et la deuxième partie de régulation de piston (142b) relie le raccord de conduite veineux (VL) au raccord de sortie (WB).

4. Dispositif de remplissage et de recirculation selon l'une quelconque des revendications précédentes, **caractérisé en ce que**, dans la position de rotation de vanne selon b., la première partie de régulation de piston (142a) relie le raccord d'entrée (B) au raccord de conduite artériel (AL).

5. Dispositif de remplissage et de recirculation selon l'une quelconque des revendications précédentes 1 à 3, **caractérisé en ce que** dans la position de rotation de vanne selon b., la première partie de régulation de piston (142a) sépare le raccord d'entrée (B) du raccord de conduite artériel (AL).

6. Procédé de réalisation d'un processus de remplissage et de recirculation d'un système de conduite côté sang d'un appareil de dialyse en utilisant un dispositif de remplissage et de recirculation selon l'une quelconque des revendications 1 à 5, comprenant les étapes suivantes :
E1. Raccorder le raccord de récipient de fluide (12) au seul raccord de fluide (11) du récipient de fluide médical (10) pour la vanne à quatre voies (14) totalement bloquée, ainsi que raccorder les parties de conduite artérielles et veineuses (2, 4) aux raccords de conduite artériel et veineux (AL, VL) de la vanne à quatre voies (14) encore bloquée,
E2. Rincer un trajet d'écoulement de fluide le long de la partie de raccord de fluide artérielle (4) et de la partie de raccord de fluide veineuse (2) de l'appareil de traitement extracorporel du sang (1) par la rotation du piston rotatif de vanne (142) à partir d'un emplacement de blocage de vanne dans la position de rotation de piston de vanne a.
E3. Faire recirculer le trajet d'écoulement de fluide en poursuivant la rotation du piston de vanne (142) à partir de la position de rotation de piston de vanne a. dans la position de rotation de piston de vanne b.
E4. Raccorder le système de conduite de fluide de l'appareil de traitement extracorporel du sang à un patient et réaliser un traitement du sang d'après la poursuite de rotation précédente du piston rotatif de vanne (142) à partir de la position de rotation de piston de vanne b., de préférence sur la position de rotation de piston de vanne d. dans la position de rotation de vanne c. et facultativement
E5. Vider le système de conduite de fluide du sang en poursuivant la rotation du piston rotatif de vanne (142) à partir de la position de rotation de piston de vanne c. dans la position de rotation de piston de vanne e.

7. Procédé de réalisation d'un processus de remplissage et de recirculation selon la revendication 6, **caractérisé en ce que**, à l'étape de procédé E4, la partie de conduite artérielle (2) est raccordée en premier au patient, pour chasser le fluide qui se trouve dans le système de conduite de fluide par le sang, puis la partie de conduite veineuse (4) est raccordée au patient.

8. Procédé de réalisation d'un processus de remplissage et de recirculation selon la revendication 6 ou 7, **caractérisé en ce qu'**à l'étape E4 la vanne à quatre voies (14) est séparée de la partie de conduite artérielle (2), après que le piston rotatif de vanne (142) a poursuivi sa rotation dans 1a position de rotation de vanne d.

9. Procédé de réalisation d'un processus de remplissage et de recirculation selon l'une quelconque des revendications 6 à 8, **caractérisé en ce qu'**à l'étape de procédé E4 1a vanne à quatre voies (14) est séparée de la partie de conduite veineuse (4), après que le piston rotatif de vanne (142) a poursuivi sa rotation dans la position de rotation de vanne c.

10. Procédé de réalisation d'un processus de remplissage et de recirculation selon la revendication 6, **caractérisé en ce qu'**à l'étape de procédé E5 la vanne à quatre voies (14) a été raccordée à nouveau au système de conduite de fluide de l'appareil de traitement extracorporel du sang, avant que le piston rotatif de vanne (142) poursuive sa rotation dans la position de rotation de vanne e.

11. Utilisation d'un dispositif de remplissage et de recirculation selon l'une quelconque des revendications 1 à 5 pour remplir un système de conduite de fluide côté sang d'un appareil de dialyse, de préférence selon un procédé selon l'une quelconque des revendications 6 à 10.
